# EUROPEAN PATENT APPLICATION

(11) **EP 2 476 408 A1**
(43) Date of publication of application: **18.07.2012**
(21) Application number: 10815324.8
(22) Date of filing: 06.09.2010
(51) Int. Cl.: A61K 8/67, A61K 31/661, A61P 17/00, A61Q 5/02, A61Q 19/02

(54) **EXTERNAL PREPARATION CONTAINING PANTETHINE PHOSPHATE ESTER**

(30) Priority: 11.09.2009 JP 2009210888
(71) Applicant: Daiichi Fine Chemical Co., Ltd., Takaoka-shi Toyama 933-8511 (JP)
(72) Inventor: WADA Koichi, Takaoka-shi Toyama 933-8511 (JP); SUMI Koichi, Takaoka-shi Toyama 933-8511 (JP); SHIMOMURA Shihoko, Takaoka-shi Toyama 933-8511 (JP)
(74) Representative: Grünecker, Kinkeldey, Stockmair & Schwanhäusser
(86) International application number: PCT/JP2010/065202
(87) International publication number: WO 2011/030727

(57) **Abstract**

A pantethine phosphate ester represented by the following formula (1) or formula (2) exhibits superior tyrosinase inhibitory effect and melanin production inhibitory effect.

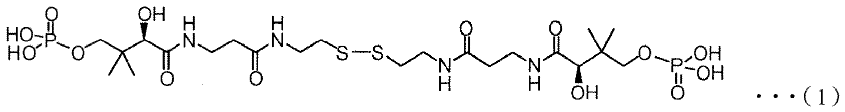

## Description

### Technical Field

The present invention relates to a topical preparation containing a pantethine phosphate ester.

### Background Art

Pantethine is a drug useful for use in (1) prevention and treatment of pantothenic acid deficiency; (2) supplement in the event where a demand for pantothenic acid increases and its intake from meals is insufficient, such as a wasting disease, hyperthyroidism, pregnant and parturient women, and lactating women; (3) prevention and treatment of hyperlipidemia, atonic constipation, and adverse drug reactions caused by streptomycin and kanamycin, improvement of acute eczema, chronic eczema, and a platelet count and bleeding tendency in a blood disease, in the case to which deficiency or metabolic disturbance of pantothenic acid presumably contributes; or the like (Non Patent Literature 1). Further, a preventive effect on diarrhea (Patent Literature 1), a Bifidobacterium proliferative effect in the intestine (Patent Literature 2), etc., have been known.

Further, the following effects have been known for pantethine used as a topical preparation:
(1) Pigmentation improving effect, whitening effect (Patent Literature 3 and Non Patent Literature 2);
(2) Therapeutic effect on hyperpigmentation disease (Patent Literature 4);
(3) Moisturizing effect (Non Patent Literature 3);
(4) Anti-acne effect (Patent Literature 5);
(5) Dermal irritation reducing effect(Patent Literature 6);
(6) Skin quality improving effect (Patent Literature 7); and
(7) Hair strength increasing effect (Patent Literature 8).

It has been known that whitening activity of pantethine is exhibited, even when it is ingested orally together with another substance having whitening effect (Patent Literature 9 and 10).

### Citation List

### Patent Literature

Patent Literature 1: JP 2006-282507 A
Patent Literature 2: JP 59-132884 A
Patent Literature 3: JP 56-73012 A
Patent Literature 4: JP 58-134022 A
Patent Literature 5: JP 58-134023 A
Patent Literature 6: JP 57-35511 A
Patent Literature 7: JP 58-128310 A
Patent Literature 8: JP 62-185007 A
Patent Literature 9: JP 2005-194203 A
Patent Literature 10: WO 2006/003965
Patent Literature 11: JP 60-64922 A
Patent Literature 12: WO 2004/028523
Patent Literature 13: JP 2007-269786 A

### Non Patent Literature

Non Patent Literature 1: Collection of Ethical Drugs (Iryou-you Iyakuhin Shu) 2009 version, p. 1900 to 1901 (2008), edited by Japan Pharmaceutical Information Center
Non Patent Literature 2: Daiichi Fine News, No. 7, p. 10 (1991)
Non Patent Literature 3: Daiichi Fine News, No. 5, p. 8 (1990)
Non Patent Literature 4: Quasi Drug Source Material Specifications 2006, Yakuji Nippo Limited, p. 1240 to 1241
Non Patent Literature 5: The Japanese Pharmacopoeia (15th Edition) Manual (Nihon Yakkyokuho (15th Edition) Kaisetsu-sho), 2006, p. C-3202 to C-3206

### Summary of Invention

### Technical Problem

It has been reported, however, that tyrosinase inhibitory activity, which is one of the mechanisms of the whitening effect of pantethine, does not rely pantethine per se, but to pantetheine derived from it by reducing a disulfide bond (Non Patent Literature 2). On the other hand, since pantetheine exhibiting the whitening activity has drawbacks such as poor stability and odor due to the presence of an SH group, it has not been able to be added into a topical preparation (Patent Literature 11). Further, the present inventors have confirmed that pantetheine is easily oxidized to pantethine in an aqueous solution at pH 5 or higher.

Calcium pantetheine-S-sulfonate is a compound, for the bad smell of pantetheine is improved by protecting the SH group of pantetheine by sulfonic acid. Although an aqueous solution containing 70% of calcium pantetheine-S-sulfonate is commercially-supplied (Non Patent Literature 4), its viscosity is high and its stability is insufficient, and the present inventors have confirmed that it has a drawback in handling.

The present invention was made in view of the problems of the conventional art with an object for providing a skin topical preparation having better whitening effect than pantethine as described above.

### Solution to Problem

The inventors conducted studies intensively to attain the object and discovered that pantethine-4',4"-diphosphate and pantethine-4'-phosphate have excellent tyrosinase activity inhibitory effect and melanin production inhibitory effect, thereby completing the present invention.

More particularly, the present invention provides a tyrosinase activity inhibitor comprising a pantethine phosphate ester represented by the following formula (1) or formula (2) or a pharmaceutically acceptable salt adduct thereof as an active ingredient. Further, it provides a melanin production inhibitor comprising a pantethine phosphate ester represented by the following formula (1) or formula (2) or a pharmaceutically acceptable salt adduct thereof as an active ingredient.

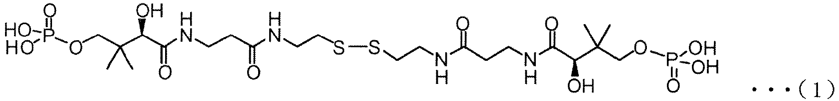

Further, the present invention provides a topical preparation comprising the tyrosinase activity inhibitor. The present invention provides a topical preparation comprising the melanin production inhibitor. The topical preparation according to the present invention is preferably a whitening skin topical preparation.

The inventors have found that by phosphorylating a hydroxy group at the 4'-position of pantethine or hydroxy groups at 2 positions of the 4'-position and the 4"-position, the whitening activity can be made higher than pantethine. Further, the inventors have found that by phosphorylating hydroxy groups at 2 positions of the 4'-position and the 4"-position of pantethine, the stability can be improved from pantethine.

Pantethine has a drawback in the stability of material properties. Pantethine exists as an amorphous powder at normal temperature when it is in an anhydrous state, but due to its high hygroscopicity, it cannot maintain the powdery state and becomes pasty. Consequently, pantethine is supplied as a viscous liquid. Pantethine according to The Japanese Pharmacopoeia is an aqueous solution containing 80% of pantethine, and is a viscous and colorless to yellowish clear liquid. However, the storage stability of the 80%-pantethine liquid is not exactly good, and a drawback has been known that it needs to be preserved at 10°C or lower (Non Patent Literature 5).

As a method for stabilizing pantethine, the following methods have been known:
(1) Method in which pantethine is mixed with an excipient (Patent Literature 12); and
(2) Method in which the pH of an aqueous solution is set to 4 to 6 (Patent Literature 13).
However, in using pantethine as a topical preparation, there are drawbacks in the method (1) that it dissolves poorly when it is added to a solution product, and in the method (2) that it cannot maintain the stability when it is added to a product with the pH of 4 or less or the pH of 6 or more.

A method of enhancing the stability by attaching a modification group, such as a phosphate group and a glycosyl group, to an easily degradable compound has been already put into practical use for vitamin C or vitamin B₆ (Hiroyuki Mima, et al., Vitamin, 41, 387-398 (1970); JP 3-139288 A; and WO 2005/033123). However, the modification position or a kind of the modification group required for enhancing the stability vary among different compounds, and can be hardly inferred. Further, the physiological activity may occasionally vary depending on a modification position or a modification group. For example, collagen production promoting activity of a vitamin C derivative in a human fibroblast varies depending on the modification position and the modification group (I. Yamamoto, et al., Journal of Nutrition (1992), 122, 871-877).

With respect to pantethine, there are 4 hydroxy groups in a molecule (2'-position, 4'-position, 2"-position, and 4"-position), and there has been no knowledge at all about: a modification at which position and with which modification group would contribute to stabilization without impairing the function. The inventors have discovered that for stabilization, modification at 4'-position or 4"-position, but not at 2'-position or 2"-position, is necessary, and that by modifying both the 4'-position and the 4"-position, the stability can be enhanced significantly.

With respect to a kind of a modification group, the inventors have confirmed that there is little difference in the stability between a phosphate ester and a glycoside and the molecule can be stable with either of them. However, the inventors have also found that the whitening activity of pantethine decreases by glycosidation, but the whitening activity of pantethine improves by phosphorylation. More particularly, in a human skin three-dimensional model test, the melanin production inhibitory activity of pantethine-4',4"-di-α-glucoside or pantethine-4",4"-di-β-galactoside decreased compared to pantethine, meanwhile the melanin production inhibitory activity of pantethine-4',4"-diphosphate improved compared to pantethine. It has become clear that the activity is even higher than sodium ascorbyl-2-phosphate, which is broadly used as a whitening agent.

Further, although mushroom tyrosinase inhibitory activity was not observed with pantethine, the inventors have made it clear hereinafter that the inhibitory activity was recognized with pantethine-4'-phosphate or pantethine-4',4"-diphosphate equivalent to β-arbutin, which is broadly used as a whitening agent.

With respect to pantethine-4',4"-diphosphate, uses as a source material for biosynthesis of coenzyme A (JP 50-126884 A) and as a synthetic intermediate to a derivative of coenzyme A (Bulletin of the Chemical Society of Japan, 49, 1122-1125 (1976)) have been reported. Further, it has been reported that pantethine-4',4"-diphosphate is a growth factor for Bifidobacterium (Z. Tamura, et al., Proc. Japan Acad., 48, 138-143 (1972)). However, no other uses have been known, and a use as a topical preparation has also not been known.

In JP 54-160315 A, a pantethine fatty acid ester (four positions of 2'-position, 4'-position, 2"-position, and 4"-position being modified) is described, teaching that the substance has a sebum secretion promoting effect, but there is no description about improvement of the stability. JP 56-73012 A discloses a whitening cosmetic containing pantethine and/or a derivative thereof added. In this regard, the pantethine derivative means a pantethine fatty acid ester (without specifying a modification position among four positions of 2'-position, 4'-position, 2"-position, and 4"-position or the number of modifications). It is described that the pantethine fatty acid ester inhibits tyrosinase of mouse melanoma under the coexistence of a mouse liver homogenate at the same level as pantethine. It is not described that the stability of the fatty acid ester is improved.

In U.S. Patent No. 4839164 is disclosed a patent of a cosmetic combination containing both pantethine or a cosmetically acceptable pantethine ester and trehalose, wherein the pantethine ester is a pantethine palmitate or a pantethine sulfonate, and not a pantethine phosphate ester. Further, the literature does not teach improvement of effect or stability by using a pantethine ester in place of pantethine. Further, a topical preparation according to the literature is limited to a mixture with trehalose, but a topical preparation according to the present invention does not necessitate inclusion of trehalose.

The present invention can provide an excellent tyrosinase activity inhibitor and a melanin production inhibitor comprising pantethine-4',4"-diphosphate, pantethine-4'-phosphate, or a pharmaceutically acceptable salt adduct thereof as an active ingredient. Pantethine-4',4"-diphosphate or pantethine-4'-phosphate according to the present invention can exhibit, unlike pantethine, as it is the tyrosinase inhibitory activity, and therefore it is effective as a whitening skin topical preparation. Since the pantethine-4',4"-diphosphate or pantethine-4'-phosphate according to the present invention improves the whitening activity of pantethine in a human skin three-dimensional model, and exhibits higher activity than an ascorbyl-2-phosphate sodium salt, which has been heretofore broadly used as a whitening agent, so that it is effective as a whitening skin topical preparation. Consequently, a topical preparation containing pantethine-4',4"-diphosphate or pantethine-4'-phosphate as a tyrosinase activity inhibitor and/or a melanin production inhibitor can be favorable utilized as a cosmetic or a drug aiming at whitening. In this connection, since pantethine-4',4"-diphosphate is stable in an aqueous solution over a wide pH range, it is especially effective from a viewpoint of use in a topical preparation.

The present invention provides further the following [1] to [6]:
[1] Use of pantethine-4',4"-diphosphate, pantethine-4'-phosphate, or a pharmaceutically acceptable salt adduct as a tyrosinase activity inhibitor;
[2] Use of pantethine-4',4"-diphosphate, pantethine-4'-phosphate, or a pharmaceutically acceptable salt adduct as a melanin production inhibitor;
[3] Use of pantethine-4',4"-diphosphate, pantethine-4'-phosphate, or a pharmaceutically acceptable salt adduct for producing a tyrosinase activity inhibitor;
[4] Use of pantethine-4',4"-diphosphate, pantethine-4'-phosphate, or a pharmaceutically acceptable salt adduct for producing a melanin production inhibitor;
[5] A method for inhibiting tyrosinase activity in a target by administering pantethine-4',4"-diphosphate, pantethine-4'-phosphate, or a pharmaceutically acceptable salt adduct to the target; and
[6] A method for inhibiting melanin production in a target by administering pantethine-4',4"-diphosphate, pantethine-4'-phosphate, or a pharmaceutically acceptable salt adduct to the target.

### Brief Description of Drawings

[Figure 1] Figure 1 is a graph showing comparisons of pH-stability among pantethine-4',4"-diphosphate, pantethine-4',4"-di-α-glucoside, pantethine, and calcium pantetheine-S-sulfonate.
[Figure 2] Figure 2 is a graph showing comparisons of pH-stability among pantethine-4',4"-diphosphate, pantethine-4'-phosphate, and pantethine.
[Figure 3] Figure 3 is a graph showing comparisons of pH-stability among pantethine, pantethine-4',4"-di-α-glucoside, and pantethine-4'-α-glucoside.
[Figure 4] Figure 4 is a graph showing comparisons of stability among pantetheine-4'-phosphate, pantetheine, and pantethine.
[Figure 5] Figure 5 is a graph showing the results of melanin formation inhibition tests in a human skin three-dimensional model.

### Description of Embodiments

Favorable embodiments of the present invention will be described in detail below.
Pantethine-4',4"-diphosphate and pantethine-4'-phosphate to be used according to the present invention are pantethine phosphate esters respectively represented by the following formula (1) or formula (2). A pharmaceutically acceptable salt adduct of the pantethine phosphate esters may be used according to the present invention.

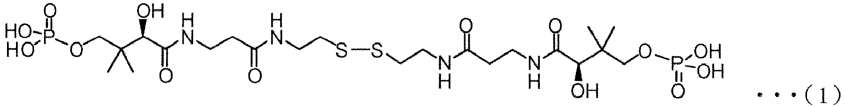

Pantethine-4',4"-diphosphate and pantethine-4'-phosphate to be used according to the present invention are heretofore known compounds. Examples of a chemical synthetic method for pantethine-4',4"-diphosphate include a synthesizing method, by which pantetheine-4'-phosphate is synthesized, and then oxidized to form a disulfide bond (Chem. Pharm. Bull., 15, 648-654 (1967)); a method of synthesizing pantethine-4',4"-diphosphate from pantothenonitrile-4'-phosphate and cysteamine, via 2-[2-(4-phosphopanto)ethyl]-2-thiazoline (JP 42-21333 B); and a method of phosphorylating pantethine by a phosphorylating reagent and then removing a protecting group (GB. Patent No. 749512, and JP 42-24884 B).

Further, pantetheine-4'-phosphate can be produced by phosphorylating pantetheine enzymatically (J. Biol. Chem., 207, 761-765 (1954)). Further, pantetheine-4'-phosphate can be obtained by hydrolyzing coenzyme A enzymatically (J. Bio. Chem., 206, 533-545 (1954)). Further, a part of a biosynthesis system of coenzyme A, in which pantetheine-4'-phosphate is biosynthesized from pantothenic acid, via 4'-phosphopantothenic acid, and 4'-phosphopantothenoyl cysteine, may be utilized. By oxidizing pantetheine-4'-phosphate obtained by the above methods, pantethine-4',4"-diphosphate can be produced.

While, pantethine-4'-phosphate can be synthesized by oxidizing pantetheine-4'-phosphate and pantetheine to form a disulfide bond. Further, it is formed as an intermediate, when pantethine is phosphorylated chemically or enzymatically to form pantethine-4',4"-diphosphate. Further, it is formed as an intermediate, when a phosphoric ester group of pantethine-4",4"-diphosphate is hydrolyzed by phosphatase, etc.

The afore-described pantethine-4',4"-diphosphate and pantethine-4'-phosphate can form a salt adduct with a pharmaceutically acceptable base, however, there occurs no difference in effect between the compounds and a salt adduct thereof, and they should be rather selected according to properties of a preparation using them. As a pharmaceutically acceptable salt forming a salt adduct with the pantethine phosphate esters represented by the above formula (1) and formula (2), an inorganic salt, such as a sodium salt, a calcium salt, a barium salt, a potassium salt, an ammonium salt, a magnesium salt, and an aluminium salt, and an organic salt, such as monoethanolamine, diethanolamine, triethanolamine, and dicyclohexylamines, can be selected. Especially preferable are a sodium salt, a calcium salt, a barium salt, and a magnesium salt.

A barium salt of pantethine-4',4"-diphosphate is powder (JP 42-24884 B). Further, the inventors have confirmed that its sodium salt, calcium salt and magnesium salt are also powder. Consequently, a salt of pantethine-4',4"-diphosphate is easy to handle compared with pantethine and calcium pantetheine-S-sulfonate, which are high viscosity aqueous solutions, and is superior as a source material for a topical preparation.

Examples of the topical preparation are not particularly restricted and may be any form of cosmetics including basic cosmetics, such as a milky lotion, a cream, a lotion, a skin lotion, a beauty essence, a pack, a cleansing preparation, and a makeup base; makeup cosmetics, such as a foundation, a blush, an eye shadow, an eyeliner, and a manicure; a dispersion; an ointment; a liquid preparation; an aerosol; a patch; a cataplasm; a liniment; hair cosmetics, such as a shampoo, a tonic, and a conditioner; a facial cleanser; a body cosmetic; a bathwater additive; a soap; a perfume; shaving-related cosmetics, such as a shaving cream, and an after-shave lotion; a deodorant; an antiperspirant; or a topical drug.

A topical preparation according to the present invention includes a pharmaceutically acceptable carrier or a cosmetically acceptable carrier in addition to the aforedescribed essential ingredient. Examples of a pharmaceutically acceptable carrier or a cosmetically acceptable carrier include various ingredients usually used in a cosmetic, a quasi drug, a topical drug, etc., namely such as water, a lower alcohol, a polyhydric alcohol, an oil solution, a surfactant, a humectant, a water-soluble polymer, a thickener, a film-forming agent, a powder, a chelating reagent, a pH adjuster, an extract derived from plants, animals and microbes, a saccharide, amino acids, organic amines, a synthetic resin emulsion, a skin nutrient preparation, vitamins, an antioxidant, an antioxidant aid, a fragrance, various medicinal agents, which may be added appropriately to the extent the advantageous effects of the present invention should not be impaired. Further, pantethine-4',4"-diphosphate and pantethine-4'-phosphate may be used together. A combination of pantethine-4',4"-diphosphate and/or pantethine-4'-phosphate and heretofore known medicinal ingredients, such as a whitening agent, an antioxidant agent, a cell activating agent, an anti-inflammatory agent, and an ultraviolet rays protective agent, forming a skin topical preparation, can further enhance the advantageous effects of the present invention by means of adding the effect of such medicinal agents on top of the action effect of pantethine-4',4"-diphosphate and/or pantethine-4'-phosphate.

The content of pantethine-4',4"-diphosphate and/or pantethine-4'-phosphate in a topical preparation is preferably 0.05 to 25 % by weight, and more preferably 0.1 to 5 % by weight. Within the range pantethine-4',4"-diphosphate and/or pantethine-4'-phosphate can be admixed stably, and the preparation is superior in tyrosinase activity inhibitory effect and melanin production inhibitory effect, exerting their high whitening effect.

### Examples

### Preparation of test compound

A pantethine-4',4"-diphosphate barium salt was synthesized according to a description of Chem. Pharm. Bull., 15, 648-654 (1967) and JP 42-21333 B. A free acid of pantethine-4',4"-diphosphate was obtained by desalting a barium salt thereof using an ion exchange resin according to JP 42-24884 B. A pantethine-4',4"-diphosphate sodium salt was obtained by salt-exchanging a pantethine-4',4"-diphosphate barium salt using an ion exchange resin. Pantethine-4'-phosphate was obtained by mixing pantetheine-4'-phosphate and pantetheine, oxidizing the mixture with hydrogen peroxide to form a mixture of pantethine-4'-phosphate, pantethine, and pantethine-4',4"-diphosphate, and purifying the same by ODS column chromatography.

### Example 1 (Comparison of pH stability of barium pantethine-4',4"-diphosphate, pantethine, and calcium pantetheine-S-sulfonate)

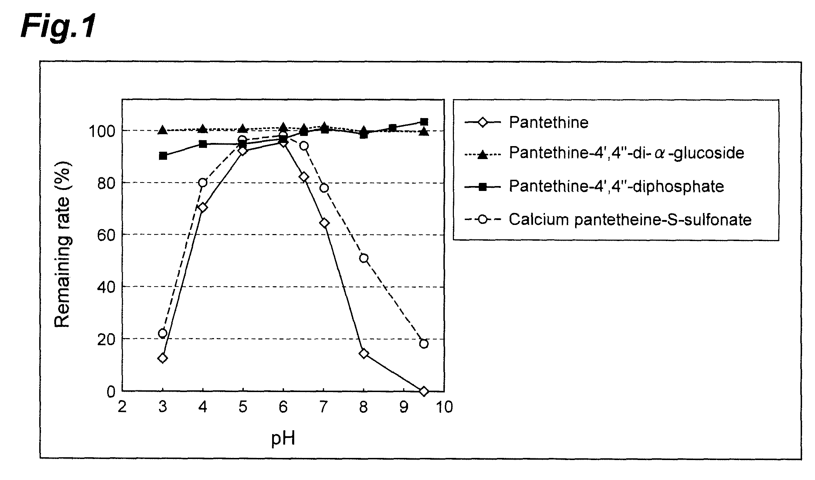

50 mM buffer solutions (pH 3 to 9.5) containing 0.1% (w/v) of barium pantethine-4',4"-diphosphate were prepared. Sodium citrate buffer solutions were used as buffer solutions at pH 3, 4, 5 and 6. Sodium phosphate buffer solutions were used as buffer solutions at pH 6.5 and 7. Tris-HCl buffer solutions were used as buffer solutions at pH 8 and 9.5. Each 0.4 mL of the solutions containing 0.1% (w/v) of barium pantethine-4',4"-diphosphate was encapsulated in a 1 mL-glass ampule and heated at 40°C for 6 months. Samples before and after the heating were analyzed by HPLC to measure the content of pantethine-4',4"-diphosphate. The same operations were carried out for pantethine and calcium pantetheine-S-sulfonate at the same concentration, and the results were compared (Figure 1).

As the result, there was no difference in stability among pantethine-4',4"-diphosphate, pantethine, and calcium pantetheine-S-sulfonate between pH 5 and 6, and all the compounds were stable; but in either pH region of pH 4 or lower, or pH 6.5 or higher, pantethine and calcium pantetheine-S-sulfonate were clearly degraded, while pantethine-4',4"-diphosphate was stable.

### Example 2 (Comparison of pH stability of pantethine-4',4"-diphosphate, pantethine-4'-phosphate and pantethine)

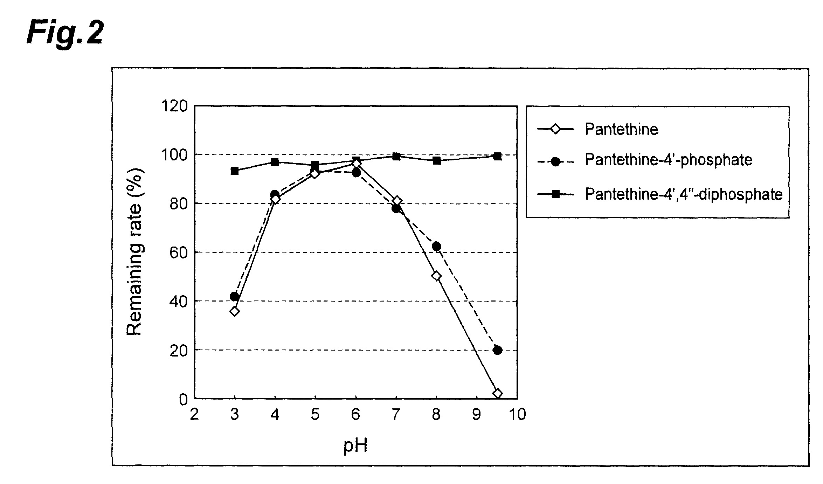

50 mM buffer solutions (pH 3 to 9.5) containing 0.1 % (w/v) of sodium pantethine-4',4"-diphosphate were prepared. Sodium citrate buffer solutions were used as buffer solutions at pH 3, 4, 5 and 6. A sodium phosphate buffer solution was used as a buffer solution at pH 7. Tris-HCl buffer solutions were used as buffer solutions at pH 8 and 9.5. Each 0.4 mL of the solutions was encapsulated in a 1 mL-glass ampule and heated at 40°C for 3 months. Samples before and after the heating were analyzed by HPLC to measure the content of pantethine-4',4"-diphosphate. The same operations were carried out for pantethine and pantethine-4'-phosphate at the same concentration, and the results were compared (Figure 2).

As the result, there was no difference in stability among pantethine, pantethine-4'-phosphate, and pantethine-4',4"-diphosphate at pHs between 5 and 6. But in a pH region of pH 4 or lower, or pH 7 or higher, pantethine was clearly degraded and pantethine-4'-phosphate exhibited similar pH stability as pantethine. On the other hand, pantethine-4',4"-diphosphate was stable.

### Example 3 (Comparison of stability of pantethine-4',4"-di-α-glucoside, pantethine-4'-α-glucoside, and pantethine)

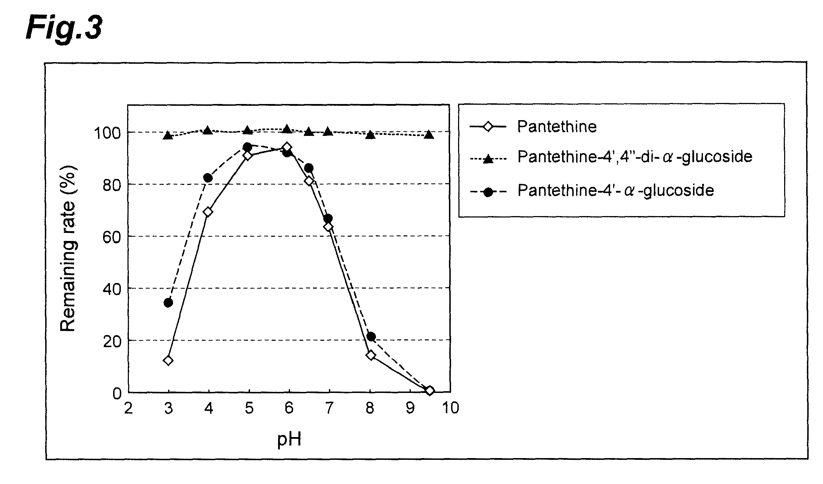

50 mM sodium citrate buffer solutions (pH 3 to 9.5) containing 0.1% (w/v) of pantethine-4',4"-di-α-glucoside were prepared. Sodium citrate buffer solutions were used as buffer solutions at pH 3, 4, 5 and 6. A sodium phosphate buffer solution was used as buffer a solution at pH 7. Tris-HCl buffer solutions were used as buffer solutions at pH 8 and 9.5. Each 0.4 mL of the solutions was encapsulated in a 1 mL-glass ampule and heated at 40°C for 6 months. Samples before and after the heating were analyzed by HPLC to measure the content of pantethine-4',4"-di-α-glucoside. The same operations were carried out for pantethine-4'-α-glucoside, and pantethine at the same concentration, and the results were compared (Figure 3).

There was no difference in stability among pantethine-4',4"-di-α-glucoside, pantethine-4'-α-glucoside, and pantethine between pH 5 and 6. But in a pH region of pH 4 or lower, or pH 6.5 or higher, pantethine and pantethine-4'-α-glucoside were clearly degraded. While pantethine-4',4"-di-α-glucoside was stable.

### Example 4 (Comparison of stability of pantetheine-4'-phosphate, pantetheine, and pantethine)

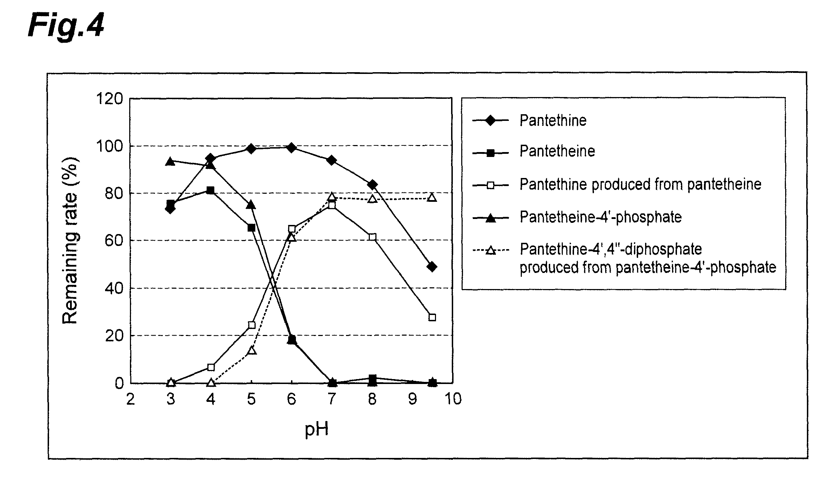

Fifty mM sodium citrate buffer solutions (pH 3, 4, 5, and 6), a 50 mM sodium phosphate buffer solution (pH 7), and 50 mM tris-HCl buffer solutions (pH 8, 9.5) containing 0.1% (w/v) of pantetheine-4'-phosphate were prepared. Each 0.4 mL of the solutions was encapsulated in a 1 mL-glass ampule and heated at 40°C for 1 month. Samples before and after the heating were analyzed by an HPLC to measure the content of pantetheine-4'-phosphate. The same operations were carried out for pantetheine at the same concentration, and the results were compared (Figure 4).

At pH 3 and 4, the stability of pantetheine-4'-phosphate was improved from pantetheine. At pH 5 or higher, pantetheine-4'-phosphate was oxidized and changed to pantethine-4',4"-diphosphate. Pantetheine was also oxidized at pH 5 or higher and changed to pantethine. At pH 7 or higher, the oxidation progressed and neither the pantetheine-4'-phosphate nor pantetheine remained.

The reason behind the decrease in the amount of pantethine produced from pantetheine at pH 8 and pH 9.5 is believed to be attributable to degradation during heating of the produced pantethine. While, the amount of pantethine-4',4"-diphosphate produced from pantetheine-4'-phosphate did not decrease even in an alkaline condition.

### Example 5 (Tyrosinase inhibition test)

As mushroom tyrosinase, Item number: T-3824 by Sigma-Aldrich, Inc., was used. As a substrate L-DOPA, Item number: 046-06564 by Wako Pure Chemical Industries, Ltd., was used. 12 mM L-DOPA (final concentration: 3.3 mM) was mixed with each sample dissolved in a 0.1 M phosphate buffer solution (pH 7.0) and the liquid volume of the solution was adjusted by a 0.1 M phosphate buffer solution (pH 7.0), to which a 500 units/mL tyrosinase solution was added (final concentration: 25 units/mL) to initiate a reaction.

After the reaction at room temperature for 1 min, the increase of dopaquinone produced from L-DOPA by tyrosinase was measured by UV absorption (492 nm). IC₅₀ defined as a sample concentration required for reducing the dopaquinone production amount by 50% with reference to the production amount in the case of no addition of a sample is shown in Table 1.

**[Table 1]**

| Sample | IC₅₀ |
|---|---|
| Pantethine | Not inhibiting |
| Pantethine-4'-phosphate | 24 mM |
| Pantethine-4',4"-diphosphate | 109 mM |
| β-Arbutin | 95 mM |

| | |
|---|---|
| * Pantethine did not exhibit inhibitory activity even at 335 mM. | |

Although pantethine did not exhibit tyrosinase inhibitory activity even at 335 mM, pantethine-4'-phosphate and pantethine-4',4"-diphosphate exhibited inhibitory activity (IC₅₀ values were 24 mM and 109 mM, respectively). IC₅₀ of β-arbutin, which is said to have tyrosinase inhibitory activity and used broadly as a whitening agent, was 95 mM and more or less equal to pantethine-4'-phosphate or pantethine-4',4"-diphosphate.

### Example 6 (Melanin production inhibition test in human skin three-dimensional model

To a horny layer side of LabCyte MELANO-MODEL (Item number: 401225 by J-TEC), which is a human skin three-dimensional model containing melanocytes, 50 µL of a PBS (-) solution of a sample was applied, and the cells were cultured under the conditions of 37°C and 5% CO₂ using an attached culture medium in a CO₂ incubator for 2 weeks. The culture medium and the applied sample solution were exchanged every 2 or 3 days. After the completion of the culture, the percentage of viable cells was measured by an MTT (methylthiazole tetrazolium) assay. Further, a melanin amount was measured.
MTT Assay: Skin cells were rinsed with a PBS and i
cubated for 3 hours in an MTT culture medium adjusted to 0.5 mg/mL. Formazan was extracted by isopropanol and the absorbance at 562 nm was measured. A test in which only a PBS(-) solution was added was used as the control, and the percentage of viable cells was determined by a proportion to the measured value of the control.
Melanin amount: The skin cells after the MTT assay were immersed in a reagent obtained by dissolving 1% SDS, 0.05 mM EDTA, and a 10 mM tris-HCl buffer solution (pH 7.2). 3 µL of a 5 mg/mL Proteinase K was added and the solution was heated in a water bath at 45°C overnight to dissolve the skin. A 30% hydrogen peroxide solution and a 500 mM sodium carbonate solution were added thereto, the solution was mixed well and heated in a water bath at 80°C for 30 min, and impurities were removed by chloroform/methanol (2/1). At the same time, melanin solutions were prepared by similar operations to prepare a calibration curve, and a melanin amount in the cultured skin cells was determined.

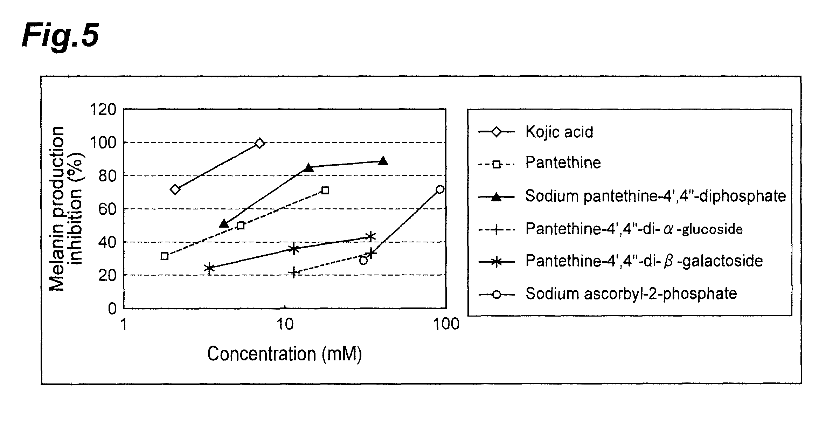

A melanin production amount in the case of 0.1% (7 mM) of kojic acid was 30% of the case where only PBS was added. When the skin cells were observed under a microscope, almost no melanocyte was recognized, and it was judged that the melanin production amount became minimal. The melanin production inhibition rate in the above case was defined as 100%, and the melanin production inhibition rate in the case of application of PBS alone was defined as 0%, and melanin production inhibition rates together with application concentrations of the respective samples are shown in Figure 5.

Concentrations at which melanin production inhibition rates reach 50% according to the plots in Figure 5 were determined be 5.3 mM for pantethine, 3.1 mM for sodium pantethine-4',4"-diphosphate, 177 mM for pantethine-4',4"-di-α-glucoside, and 77 mM for pantethine-4',4"-di-β-galactoside. Namely, if pantethine was phosphorylated, the whitening activity was strengthened 1.7-fold, but if it was converted into a glycoside, the whitening activity was weakened. Meanwhile, a melanin production 50% inhibition concentration of sodium ascorbyl-2-phosphate with an established reputation as a whitening agent was 54 mM, and the whitening activity of sodium pantethine-4',4"-diphosphate was 17 times higher than that of sodium ascorbyl-2-phosphate.

Within the application range of this test, the percentage of viable cells determined by the MTT assay was 80% or higher using any of the samples, and it was confirmed that the range has little influence on the cell viability.

Topical preparations were formulated as described in Examples 7 to 10.

### Example 7 (Lotion)

0.1 g of a ceramide composition, 2.5 g of 1,3-butylene glycol, 2.5 g of dipropylene glycol, and 0.01 g of methylparaben were heated at 80°C and stirred until it became transparent. After cooling down to 35°C, 1 g of sodium pantethine-4',4"-diphosphate, 1.0 g of sorbitol-fermented polysaccharide, and 90 mL of purified water were added with stirring, and the mixture was further stirred until dissolution, then adjusted pH to 6.4 with a 10% citrate aqueous solution, and then adjusted to 100 mL with purified water to complete a lotion.

### Example 8 (Milky lotion)

1 g of polyoxyethylene (10 EO) sorbitan monostearate, 1 g of glyceryl monostearate, 0.5 g of stearic acid, 0.5 g of behenyl alcohol, 0.1 g of a carboxy vinyl polymer, 5 g of ethyl alcohol, and 80 mL of purified water were mixed under heating at 70°C, and emulsified homogeneously. After cooling down to 40°C, 1 g of sodium pantethine-4',4"-diphosphate was added and mixed, the pH was adjusted to 7, and then the mixture was adjusted to 100 g with purified water to complete a milky lotion.

### Example 9 (Shampoo)

10.0 g of sodium methyl cocoyl taurate, 20.0 g of sodium polyoxyethylene alkyl ether sulfate, 10.0 g of lauryl dimethylaminoacetic acid betaine, 4.0 g of coconut oil fatty acid diethanolamide, 2.0 g of propylene glycol, and 0.01 g of methyl parahydroxybenzoate were added into 40 mL of purified water and the mixture was heated to 70°C for dissolution. After cooling down to 35°C, 1 g of sodium pantethine-4',4"-diphosphate was added and mixed, and the pH was adjusted to 6.8 with a 10% citrate aqueous solution and then the volume was adjusted to 100 mL with purified water to complete a shampoo.

### Example 10 (Creme)

6 g of stearyl alcohol, 2 g of stearic acid, 4 g of hydrogenated lanolin, 9 g of squalane, 10 g of octyl dodecanol, 3 g of polyoxyethylene (25 EO) cetyl alcohol ether, 2 g of glycerol monostearate, and 6 g of 1,3-butylene glycol were added into purified water 40 g and the mixture was heated to 75°C to a suspension liquid. After cooling down to 50°C, 1 g of sodium pantethine-4',4"-diphosphate was added and mixed, and the pH was adjusted to pH 7 with 6 N caustic soda, and then the volume was adjusted to 100 g with purified water to complete a cream.

All the topical preparations prepared in Examples 7 to 10 were excellent topical preparations making skin white and smooth when applied to skin. Further, there was no recognizable precipitate or the like in the topical preparations demonstrating good stability.

## Claims

1. A tyrosinase activity inhibitor comprising a pantethine phosphate ester represented by the following formula (1) or formula (2) or a pharmaceutically acceptable salt adduct thereof as an active ingredient.

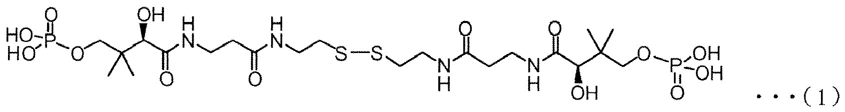

2. A melanin production inhibitor comprising a pantethine phosphate ester represented by the following formula (1) or formula (2) or a pharmaceutically acceptable salt adduct thereof as an active ingredient.

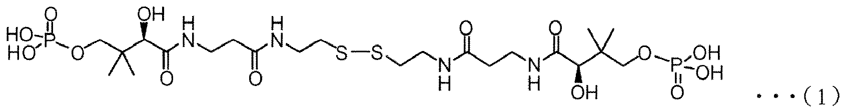

3. A topical preparation comprising the tyrosinase activity inhibitor according to claim 1.

4. A topical preparation comprising the melanin production inhibitor according to claim 2.

5. The topical preparation according to claim 3 or 4, which is a whitening skin topical preparation.
